# EUROPÄISCHE PATENTANMELDUNG

(11) **EP 4 818 073 A2**
(43) Veröffentlichungstag der Anmeldung: **07.10.2026**
(21) Anmeldenummer: 26200989.7
(22) Anmeldetag: 09.02.2021
(51) Int. Cl.: B65D 77/04

(54) **FLÜSSIGKEITSSPENDER MIT SICHERUNG**

(62) Dokumentnummer(n) der früheren Anmeldung(en) nach Art. 76 EPÜ: 21155900.0 / 4 039 235
(71) Anmelder: Aptar Radolfzell GmbH, 78315 Radolfzell (DE)
(72) Erfinder: Heinzle, Volker, 72505 Krauchenwies (DE); Trinkner, Julian, 78269 Volkertshausen (DE)
(74) Vertreter: Witte, Weller und Partner Patentanwälte mbB Stuttgart

(57) **Zusammenfassung**

Bekannt ist ein Flüssigkeitsspender zum Austrag pharmazeutischer Flüssigkeiten einschließlich Tropfenspendern, deren Flüssigkeitsspeicher durch einen Quetschflaschenkörper gebildet wird.

Erfindungsgemäß werden zwei Maßnahmen zur Sicherung solcher Flüssigkeitsspender vorgeschlagen, nämlich das Vorsehen eines Blendenkörpers (80), mittels dessen eine Kraftbeaufschlagung des Quetschflaschenkörper (20) verhindert werden kann, sowie die Gestaltung einer Kappe (140) mit einem drehbaren Verriegelungsring (146). Das Abnehmen der Kappe (140) sowie die Überführung des Blendenkörpers (80) in eine Nutzstellung erfordert gemäß bevorzugten Gestaltungen die Verformung eines ringförmigen Teilelements (90, 146).

## Beschreibung

### ANWENDUNGSGEBIET UND STAND DER TECHNIK

Die Erfindung betrifft einen Flüssigkeitsspender zur Abgabe pharmazeutischer Flüssigkeiten. Insbesondere betrifft die Erfindung Flüssigkeitsspender, die zur Abgabe von Flüssigkeiten in Form von Einzeltropfen vorgesehen sind, beispielsweise zur Applikation als Augentropfen in die Augen des Patienten.

Ein gattungsgemäßer Flüssigkeitsspender weist vorzugsweise einen Quetschflaschenkörper auf, der den Flüssigkeitsspeicher bildet. Die darin enthaltene Flüssigkeit kann durch beidseitige Kraftbeaufschlagung ausgetragen werden kann. Hierfür ist an einem Behälterhals des Quetschflaschenkörpers ein Austragkopf befestigt, der über eine Austragöffnung verfügt.

Die von der Erfindung betroffenen Flüssigkeitsspender beinhalten üblicherweise pharmazeutische Flüssigkeiten, deren Einnahme durch Kinder gesundheitlich nachteilig sein könnte. Dies ist insbesondere bei Quetschflaschenspendern problematisch, da ein Austrag hier selbst durch Kleinkinder zu bewerkstelligen ist, die ungezielt den Quetschflaschenkörper zusammendrücken und dadurch einen Flüssigkeitsaustrag bewirken.

### AUFGABE UND LÖSUNG

Aufgabe der Erfindung ist es, einen Flüssigkeitsspender dahingehend weiterzubilden, dass die Gefahr eines ungewollten Flüssigkeitsaustrags vermindert wird.

Gemäß einem ersten Aspekt der Erfindung wird ein Flüssigkeitsspender vorgeschlagen, der einen Quetschflaschenkörper mit einem Behälterhals aufweist, wobei am Behälterhals ein Austragkopf befestigt ist, der über eine Austragöffnung verfügt, durch die hindurch Flüssigkeit aus dem Quetschflaschenkörper austragbar ist. Vorzugsweise handelt es sich bei dem Spender um einen Tropfenspender zur Abgabe von Einzeltropfen, insbesondere zur ophthalmischen Anwendung. Der Austragkopf eines solchen Tropfenspenders verfügt vorzugsweise über Tropfenbildungsmittel, insbesondere in Form einer planen oder konischen Tropfenbildungsfläche, an der sich ausgetragene Flüssigkeit sammelt, bis sie sich als Tropfen von der Tropfenbildungsfläche löst. Ein solcher Tropfenspender weist vorzugsweise ein der Austragöffnung vorgeschaltetes Auslassventil auf, insbesondere wenn die enthaltene Flüssigkeit konservierungsmittelfrei ist.

Um das ungewollte Zusammendrücken des Quetschflaschenkörpers zu verhindern, insbesondere wenn Kleinkinder dies versuchen, weist der Flüssigkeitsspender gemäß dem ersten Aspekt der Erfindung einen ersten Blendenkörper auf, der seinerseits mindestens eine Sperrwandung aufweist und mindestens eine Betätigungsaussparung definiert. Dieser erste Blendenkörper ist unverlierbar und zwischen einer Sperrstellung und einer Freigabestellung beweglich am Flüssigkeitsspender vorgesehen und hierdurch gegenüber dem Quetschflaschenkörper verlagerbar.

In der Sperrstellung überdeckt der erste Blendenkörper mittels der Sperrwandung einen Teilbereich des Quetschflaschenkörpers, so dass ein Zusammendrücken des Quetschflaschenkörpers verhindert oder zumindest erschwert ist. In der Freigabestellung gibt der erste Blendenkörper mittels der Betätigungsaussparung den genannten Teilbereich des Quetschflaschenkörpers frei, so dass ein Zusammendrücken des Quetschflaschenkörpers ermöglicht ist.

Um den Flüssigkeitsspender zu verwenden, verlagert der Nutzer den Blendenkörper in die Freigabestellung. Nach Abschluss des Austragvorgangs verlagert er den Blendenkörper zurück in die Sperrstellung, in der ein ungewollter Flüssigkeitsaustrag unterbunden ist.

Der Quetschflaschenkörper kann durch seine Formgebung oder Wandungsgestaltung eine umfänglich uneinheitliche Eindrückbarkeit aufweisen. In einem solchen Fall ist der mindestens eine durch die Sperrwandung überdeckbare Teilbereich ein Flächenabschnitt, der ein besonders leichtes Eindrücken gestattet. Wenn sich der Blendenkörper in seiner Sperrstellung befindet, ist das Zusammendrücken des Quetschflaschenkörpers dadurch erschwert, dass nur ein schwerer oder ein nicht eindrückbarer Teilbereich des Quetschflaschenkörpers zugänglich ist.

Vorzugsweise ist zusätzlich ein zweiter Blendenkörper vorgesehen. Dieser weist ebenfalls mindestens eine Sperrwandung auf und definiert mindestens eine Betätigungsaussparung. Der erste Blendenkörper ist gegenüber diesem zweiten Blendenkörper verlagerbar, so dass in der Freigabestellung die Betätigungsaussparungen derart überlappen oder sogar deckungsgleich sind, dass durch beide Betätigungsaussparungen hindurch eine Kraftbeaufschlagung des Quetschflaschenkörpers ermöglicht ist. Ist der ersten Blendenkörper dagegen in der Sperrstellung, so sind die Betätigungsaussparungen derart gegeneinander versetzt, dass eine Betätigung des Quetschflaschenkörpers verhindert oder zumindest erschwert ist.

Die Sperrabschnitte beider Blendenkörper können in der Sperrstellung insbesondere mittels ihrer Sperrabschnitte eine Mantelwandung des Quetschflaschenkörpers umfänglich vollständig oder nahezu vollständig (>80%) umgeben.

Die Blendenkörper sind vorzugsweise als den Quetschflaschenkörper umgebende Schalenkörper ausgebildet.

Der erste Blendenkörper ist vorzugsweise drehbar gegenüber dem Quetschflaschenkörper ausgebildet, insbesondere um eine Hauptachse, in der auch die Austragöffnung liegt, und/oder um eine Mittelachse des Quetschflaschenkörpers. Der zweite Blendenkörper ist vorzugsweise drehfest zum Quetschflaschenkörper an diesem angebracht, insbesondere vorzugsweise mittels einer Klemmverbindung. Es ist jedoch auch eine Gestaltung von der Erfindung umfasst, bei der beide Blendenkörper gegenüber dem Quetschflaschenkörper und gegeneinander beweglich und insbesondere drehbar sind.

Der erste und/oder der zweite Blendenkörper weisen vorzugsweise einen im wesentlichen rotationssymmetrischen Hülsenabschnitt auf, in dem die mindestens eine Betätigungsaussparung in Form einer Durchbrechung vorgesehen ist. Die Hülsenabschnitte weisen vorzugsweise eine Länge auf, die es ihnen gestattet, vom oberen Ende des Quetschflaschenkörpers bis zu dessen Boden zu ragen. Die mindestens eine Durchbrechung ist als allseitig vom jeweiligen Hülsenabschnitt umgebene Aussparung ausgebildet.

Eine mögliche Gestaltung sieht vor, dass genau eine Durchbrechung am ersten bzw. am zweiten Blendenkörper vorgesehen ist. Zum Zusammendrücken übt der Nutzer mit mindestens einem Finger durch die Durchbrechung hindurch unmittelbar eine Kraft auf den Quetschflaschenkörper aus. Gegenüberliegend zu der einen Durchbrechung ist keine weitere Durchbrechung vorgesehen, so dass hier unmittelbar auf den Hülsenabschnitt und nur mittelbar auf den Quetschflaschenkörper durch den Nutzer eine Kraft ausgeübt wird. Bei einer solchen Gestaltung mit nur einer Durchbrechung am ersten und gegebenenfalls am zweiten Blendenkörper überspannt diese Durchbrechung in Umfangsrichtung vorzugsweise einen Winkelbereich zwischen 120° bis 240°, insbesondere vorzugsweise einen Winkelbereich zwischen 150° und 210°.

Bevorzugt ist eine Gestaltung, bei der einander gegenüberliegend zwei Durchbrechungen am ersten bzw. am zweiten Blendenkörper vorgesehen sind. Zum Zusammendrücken des Quetschflaschenkörpers übt der Nutzer mit jeweils mindestens einem Finger, insbesondere mit Zeigefinger und Daumen, durch die einander gegenüberliegenden Durchbrechung hindurch beidseitig ein Kraft auf den Quetschflaschenkörper aus. Bei einer solchen Gestaltung ist vorzugsweise vorgesehen, dass die beiden Durchbrechungen jeweils einen Winkelbereich zwischen 60° und 170° überspannen, vorzugsweise jeweils einen Winkelbereich zwischen 90° und 160°.

Finden zwei Blendenkörper Verwendung, so sind diese vorzugsweise in Art einer Innenschale und einer Außenschale den Quetschflaschenspeicher umgebend angeordnet, wobei eine einfache Gestaltung vorsieht, dass der erste Blendenkörper außenliegend angeordnet ist und der zweite Blendenkörper innenliegend angeordnet und vorzugsweise drehfest am Quetschflaschenkörper vorgesehen ist. Die Sperrwandung des zweiten Blendenkörpers ist dadurch im gesperrten Zustand zwischen dem Quetschflaschenkörper und der Sperrwandung des ersten Blendenkörpers angeordnet.

Es ist jedoch auch eine Bauform möglich, bei der der zweite vorzugsweise ortsfest zum Quetschflaschenkörper vorgesehene Blendenkörper die Außenschale bildet und der erste bewegliche und vorzugsweise drehbare Blendenkörper die Innenschale bildet.

Der Quetschflaschenkörper, der erste Blendenkörper und gegebenenfalls der zweite Blendenkörper sind vorzugsweise als jeweils einstückige Bauteile aus Kunststoff gefertigt. Zur Montage wird vorzugsweise der erste und gegebenenfalls der zweite Blendenkörper von oben oder vorzugsweise von unten auf den Quetschflaschenkörper geschoben. Vorzugsweise weist der erste und/oder der zweite Blendenkörper Rastmittel auf, um axial am anderen Blendenkörper oder am Quetschflaschenspeicher oder dem Austragkopf zu verrasten.

Die Blendenkörper können mindestens einen auslenkbaren Sicherungsabschnitt aufweisen, der beim Aufschieben des Blendenkörpers elastisch aufgeweitet wird und in der Endlage wieder zumindest teilweise entspannt. Insbesondere kann ein solcher elastisch auslenkbarer Sicherungsabschnitt am zweiten Blendenkörper vorgesehen sein, um nach Aufschieben des zweiten Blendenkörpers auf den Quetschflaschenkörper ein Herausziehen des Quetschflaschenkörpers aus dem zweiten Blendenkörper zu verhindern.

Insbesondere vorzugsweise weist der erste Blendenkörper eine Stützfläche auf, durch die nach Aufschieben des ersten Blendenkörpers auf den zweiten Blendenkörper im Zuge der Montage die elastische Auslenkbarkeit des Sicherungsabschnitts des zweiten Blendenkörpers beschränkt ist. Der erste Blendenkörper sichert demzufolge den zweiten Blendenkörper am Quetschflaschenkörper.

Es kann insbesondere auch eine Mehrzahl von radial auslenkbaren Sicherungsabschnitten vorgesehen sein. Vorzugsweise weist mindestens einer der Blendenkörper zwei solche Sicherungsabschnitte auf, wobei der Quetschflaschenkörper bei der Montage diese Sicherungsabschnitte voneinander weg drückt, um hindurch zu passen. Bei einer Gestaltung mit mehreren Sicherungsabschnitten ist vorzugsweise vorgesehen, dass eine gemeinsame und vorzugsweise umlaufende Stützfläche vorgesehen ist, deren radiale Auslenkbarkeit nach Abschluss der Montage beschränkt ist.

Es ist von Vorteil, wenn der erste Blendenkörper in mindestens einer der Stellungen, also der Sperrstellung und der Freigabestellung, verriegelbar ist. Zwar ist auch eine Gestaltung denkbar, bei der durch einen hohen Drehwiderstand die jeweilige Stellung lediglich reibschlüssig gehalten wird. Vorzugsweise ist jedoch eine schaltbare Verriegelung vorgesehen, mittels derer der erste Blendenkörper rotativ am Quetschflaschenkörper, am zweiten Blendenkörper oder am Austragkopf verriegelt werden kann. Diese Verriegelung erfolgt vorzugsweise durch formschlüssig wirkende Verriegelungselemente, die insbesondere einer Drehbewegung entgegenwirken können. Befinden sind die Verriegelungselemente im Eingriff miteinander, so ist eine Bewegung des ersten Blendenkörpers nicht möglich. Die Verriegelungselemente sind derart angeordnet, dass ein solcher verriegelter Zustand in der Sperrstellung oder in der Freigabestellung oder in beiden Stellungen möglich ist.

Um die Verriegelungselemente außer Eingriff zu bringen, wird vorzugsweise einer der Körper, insbesondere der erste Blendenkörper, elastisch verformt. Insbesondere kann hierfür vorgesehen sein, dass einer der Blendenkörper ein elastisch verformbares umlaufendes Ringsegment aufweist, an welchem einander gegenüber außenliegende Entriegelungsflächen vorgesehen sind und an welchem innenliegend ein in Umfangsrichtung zwischen den Entriegelungsflächen angeordnetes Verriegelungselement vorgesehen ist. Durch aufeinander zu gerichtete Kraftbeaufschlagung der Entriegelungsflächen wird das Ringsegment zusammengedrückt und das Verriegelungselement nach außen ausgelenkt, wodurch der Eingriff mit einem innenliegenden Verriegelungselement, insbesondere am zweiten Blendenkörper, gelöst wird.

Auch kann vorgesehen sein, dass am ersten Blendenkörper oder am zweiten Blendenkörper ein isoliert elastisch auslenkbares Verriegelungselement vorgesehen ist, welches durch manuelle Kraftbeaufschlagung verlagerbar ist, insbesondere verschwenkbar. Diese Verlagerung gestattet es dem auslenkbaren Verriegelungselement außer Eingriff von einem korrespondierenden Verriegelungselement am anderen Blendenkörper oder am Quetschflaschenkörper zu gelangen.

Da es insbesondere bevorzugt ist, dass die Blendenkörper zur Erzielung des Sperrzustandes bzw. des Freigabezustandes gegeneinander verdreht werden, kann es von Vorteil sein, wenn mindestens an einem der Blendenkörper hierfür eine Grifffläche vorgesehen ist. Insbesondere vorteilhaft ist eine Gestaltung, bei der der zweite Blendenkörper eine zumindest annähernd umlaufende Grifffläche aufweist. Diese ist vorzugsweise mit einer Strukturierung versehen, insbesondere mit einem regelmäßigen Muster von Erhöhungen oder Vertiefungen. Die Grifffläche ist vorzugsweise außenseitig an den oben beschriebenen auslenkbaren Sicherungsabschnitten vorgesehen.

Ein zweiter Aspekt der Erfindung, der in Anspruch 1 definiert ist, betrifft einen Flüssigkeitsspender mit einer Austragvorrichtung und einer Kappe. Der Flüssigkeitsspender verfügt über einen Flüssigkeitsspeicher, insbesondere gebildet durch einen Quetschflaschenkörper, und über einen Austragkopf mit Austragöffnung. Die Kappe überdeckt die Austragöffnung und ist zum Zwecke der Abgabe von Flüssigkeit abnehmbar und nach Nutzung wiederaufsetzbar.

Die Kappe weist einen Kappenkörper und einen am Kappenkörper drehbar angebrachten Verriegelungsring auf. An der Austragvorrichtung, insbesondere am Austragkopf, sowie am Verriegelungsring sind im aufgesetzten Zustand aufeinander zuweisende Verriegelungsprofile vorgesehen. Diese verhindern in mindestens einer Sicherungs-Drehstellung des Verriegelungsrings formschlüssig ein Abnehmen der Kappe. Die Verriegelungsprofile sind derart ausgebildet, dass ein Abnehmen der Kappe nur möglich ist, wenn der Verriegelungsring zur Austragvorrichtung in eine Freigabe-Drehstellung gedreht worden ist.

Der Verriegelungsring ist unverlierbar mit dem Kappenkörper verbunden. Vorzugsweise verfügen der Kappenkörper und der Verriegelungsring über zusammenwirkende Rastmittel, mittels derer der Kappenkörper und der Verriegelungsring unter Gewährleistung der Drehbeweglichkeit verrastbar sind, wenn der Kappenkörper im Zuge der Montage von unten in den Verriegelungsring eingeschoben wird.

Der Verriegelungsring und der Kappenkörper sich im Lieferzustand vorzugsweise drehgesichert miteinander verbunden, so dass der Zustand der Drehbarkeit erst hergestellt werden muss, insbesondere durch ein Verformen oder Entfernen eines Teilabschnitts des Verriegelungsring oder des Kappenkörpers. Insbesondere kann dieser drehsichernde Teilabschnitt bestimmungsgemäß irreversibel entfernt, bspw. abgebrochen werden. Er stellt gleichzeitig auch einen Originalitätsabschnitt dar, dessen Entfernen signalisiert, dass der Flüssigkeitsspender bereits in Betrieb genommen worden ist.

Bei einer möglichen Ausgestaltung ist es möglich, die Kappe unmittelbar nach Drehen des Verriegelungsrings in die Freigabestellung abzuziehen. In der Freigabestellung leistet der Verriegelungsring bei einer solchen Gestaltung keinen Widerstand gegen das Abziehen der Kappe.

Vorzugsweise ist jedoch vorgesehen, dass die aufeinander zuweisende Verriegelungsprofile derart ausgebildet sind, dass ein Abnehmen der Kappe in der Freigabe-DrehstellungdesVerriegelungsrings nicht unmittelbar möglich ist, sondern erst wenn zusätzlich der Verriegelungsring oder ein Teilabschnitt der Austragvorrichtung elastisch verformt wird.

Der Nutzer muss also zur Abnahme der Kappe den Verriegelungsring drehen und dann verformen, insbesondere zusammendrücken, um die Kappe zu lösen. Für einen Erwachsenen stellt dies keine relevante Erschwerung dar, da ein Drehen und Zusammendrücken des Verriegelungsrings und ein Abziehen der Kappe in einem Handgriff erfolgen kann. Für ein Kleinkind ist es hingegen schwer zu verstehen, welche Teilbewegungen erforderlich sind. Zudem kann die erforderliche Verformung des Verriegelungsrings bei geeigneter Auslegung die Kraft eines Kleinkindes überschreiten.

Insbesondere kann vorgesehen sein, dass der Verriegelungsring auf einander gegenüberliegenden Seiten Freigabe-Betätigungsflächen aufweist. Werden diese in der Freigabe-Drehstellung radial aufeinander zu gedrückt, so wird das Verriegelungsprofil am Verriegelungsring derart radial nach außen verlagert, dass anschließend ein Abnehmen der Kappe möglich ist, insbesondere ein einfaches Abziehen.

Bevorzugt wird eine Gestaltung, bei der die Austragvorrichtung und der Verriegelungsring Ausrichtungsmarkierungen aufweisen, die übereinstimmend ausgerichtet sind, wenn der Verriegelungsring sich in seiner Freigabestellung befindet. Es ist hierdurch einfach ersichtlich, ob die Kappe in der aktuellen Stellung des Verriegelungsrings verriegelt oder abnehmbar ist.

Insbesondere vorzugsweise ist die Ausrichtungsmarkierung an der Austragvorrichtung derart angeordnet, dass sie vom Verriegelungsring verdeckt wird, wenn sich dieser nicht in der Freigabestellung befindet. Dabei kann zusätzlich im Verriegelungsring eine Aussparung vorgesehen sein, durch die hindurch die Ausrichtungsmarkierung der Austragvorrichtung sichtbar ist, wenn sich der Verriegelungsring in der Freigabestellung befindet.

Wie bereits dargestellt, ist der Flüssigkeitsspender gemäß beiden Aspekten der Erfindung vorzugsweise als Tropfenspender zur Abgabe von Einzeltropfen ausgebildet, insbesondere als Tropfenspender zur ophthalmischen Anwendung. In der Ausgestaltung als Tropfenspender weist der Flüssigkeitsspender vorzugsweise Tropfenbildungsmittel auf, insbesondere in Form einer planen oder konischen Tropfenbildungsfläche.

Der Flüssigkeitsspeicher eines erfindungsgemäßen Flüssigkeitsspenders, der insbesondere durch den beschriebenen Quetschflaschenkörper gebildet wird, weist vorzugsweise ein Aufnahmevolumen zwischen 2 ml und 100 ml auf, insbesondere vorzugsweise zwischen 4 ml und 15 ml.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1 zeigt ein erstes Ausführungsbeispiel eines erfindungsgemäßen Spenders.
Fig. 2A und 2B zeigen den Vorgang der Überführung des Spenders aus seinem Sperrzustand in seinen Freigabezustand.
Fig. 3A und 3B zeigen zwei Blendenkörper des Spenders.
Fig. 3C zeigt die Blendenkörper im montierten Zustand.
Fig. 4A bis 4C verdeutlichen die Montage des Spenders.
Fig. 5A und 5B sowie 6 zeigen ein zweites Ausführungsbeispiel eines erfindungsgemäßen Spenders.
Fig. 7A und 7B zeigen die Kappe des Spenders gemäß dem zweiten Ausführungsbeispiel.
Fig. 8 zeigt den Austragkopf des Spenders gemäß dem zweiten Ausführungsbeispiel bei abgenommener Kappe.

### DETAILLIERTE BESCHREIBUNG DER AUSFÜHRUNGSBEISPIELE

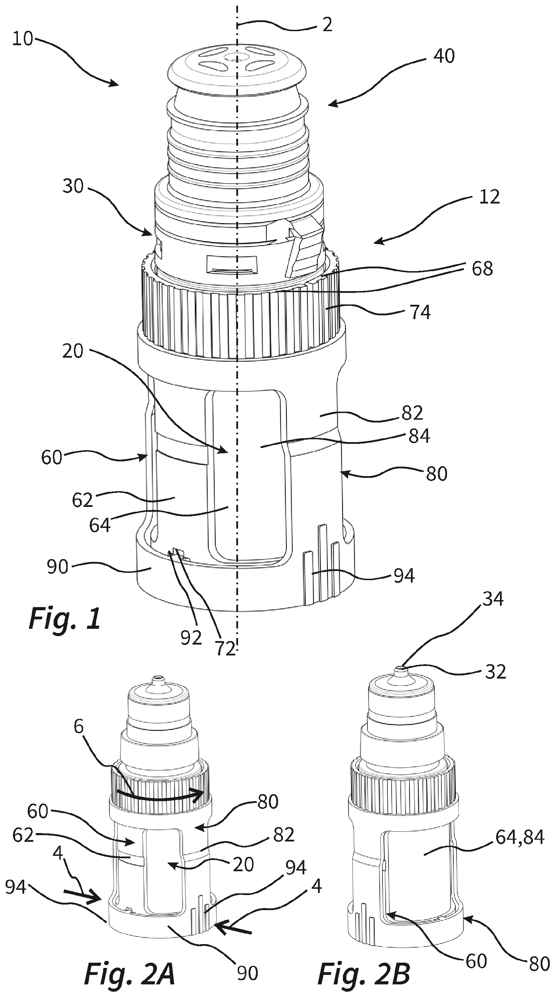

Fig. 1 zeigt einen Flüssigkeitsspender 10, der zum Austrag von Augentropfen dient, aber auch für andere pharmazeutische Anwendungen, insbesondere per Tropfenabgabe, ausgebildet sein kann.

Der Flüssigkeitsspender umfasst eine Austragvorrichtung 12 mit Flüssigkeitsspeicher in Form eines Quetschflaschenkörpers 20 und einen Austragkopf 30 sowie eine Kappe 40. Die Kappe 40 überdeckt eine Austragöffnung 32 sowie eine Tropfenbildungsfläche 34 am Austragkopf 30. Nach erstmaligem Abnehmen kann die Kappe 40 zum Zwecke der Nutzung des Flüssigkeitsspenders wiederholt abgenommen und aufgesetzt werden.

In nicht näher dargestellter Weise umfasst der Austragkopf 30 zusätzlich ein Auslassventil, welches durch Druckbeaufschlagung von Flüssigkeit im Quetschflaschenkörper 20 geöffnet werden kann, so dass Flüssigkeit zur Austragöffnung strömen kann und sich in Überkopflage der Austragvorrichtung 12 an der Tropfenbildungsfläche sammelt, bis sich der Tropfen löst.

Um eine einfache Handhabung und Dosierbarkeit zu gewährleisten, ist der Quetschflaschenkörper 20 bereits mit geringem Kraftaufwand zusammendrückbar. Dies geht jedoch mit der Gefahr einher, dass der Quetschflaschenkörper 20 ungewollt zusammengedrückt wird, beispielsweise in einer Tasche oder im Reisegepäck oder durch ein Kleinkind.

Um dies zu verhindern, ist eine Sicherung vorgesehen, die primär durch einen ersten Blendenkörper 80 und einen zweiten Blendenkörper 60 gebildet wird. Die beiden Blendenkörper 60, 80 umgeben im montierten Zustand der Fig. 1 den Quetschflaschenkörper 20. Beide Blendenkörper 60, 80 weisen eine grundsätzlich hülsenförmige oder in etwa zylindrische Form auf, wobei eine jeweilige Mantelfläche eine Sperrwandung 82, 62 bildet, die mit einander gegenüberliegenden Durchbrechungen 64, 84 versehen ist.

Mindestens einer der Blendenkörper 60, 80 ist gegenüber dem Quetschflaschenkörper 20 und gegenüber dem anderen Blendenkörper 60, 80 um die Mittelachse 2 drehbar ausgebildet, so dass je nach Relativdrehstellung der Blendenkörper 60, 80 zueinander die jeweiligen Durchbrechungen 64, 84 miteinander fluchten oder zueinander versetzt sind.

Fig. 2A zeigt den Zustand, in dem die Durchbrechungen 64, 84 zueinander versetzt sind und dadurch ein gesicherter Zustand der Austragvorrichtung 12 erzielt ist. Zwar schützen die Sperrwandungen 82, 62 bei der dargestellten Gestaltung aufgrund der Größe der Durchbrechungen 64, 84 den Quetschflaschenkörper 20 nicht umfänglich, die verbleibenden Öffnungen sind jedoch nicht groß genug, dass der Quetschflaschenkörper 20 ungewollt in relevantem Maße eingedrückt und hierdurch Flüssigkeit abgegeben werden kann.

Um ausgehend vom gesicherten Zustand der Fig. 2A einen Nutzzustand der Fig. 2B zu erzielen, müssen die Blendenkörper 60, 80 gegeneinander verdreht werden. Dies ist jedoch nicht allein mit einer einfachen Momentenbeaufschlagung möglich, da eine schaltbare Verriegelung 50 vorgesehen ist, die im verriegelten Zustand eine Drehbewegung formschlüssig unterbindet. Diese Verriegelung umfasst hierfür nach innen hervorragendes Verriegelungselemente 92 am ersten Blendenkörper 80, welche im verriegelten Zustand mit Verriegelungselementen 72 in Form von Wandungen von Vertiefungen am zweiten Blendenkörper 60 im Eingriff steht.

Um diese Verriegelung zu lösen muss ein den ersten Blendenkörper 80 unten abschließendes Ringsegment 90 verformt werden. Dieses weist hierfür einander gegenüberliegend Entriegelungsflächen 94 auf. Wird auf diese in Richtung der Pfeile 4 eine Kraft ausgeübt, so wird das Ringsegment 90 verformt und die Verriegelungselemente 92 werden radial nach außen verlagert, so dass der Eingriff mit der Verriegelungselementen 72 des zweiten Blendenkörpers 60 gelöst wird. In diesem entriegelten Zustand ist nun die Drehbewegung in Richtung des Pfeils 6 möglich, so dass der Nutzzustand der Fig. 2B erzielt ist.

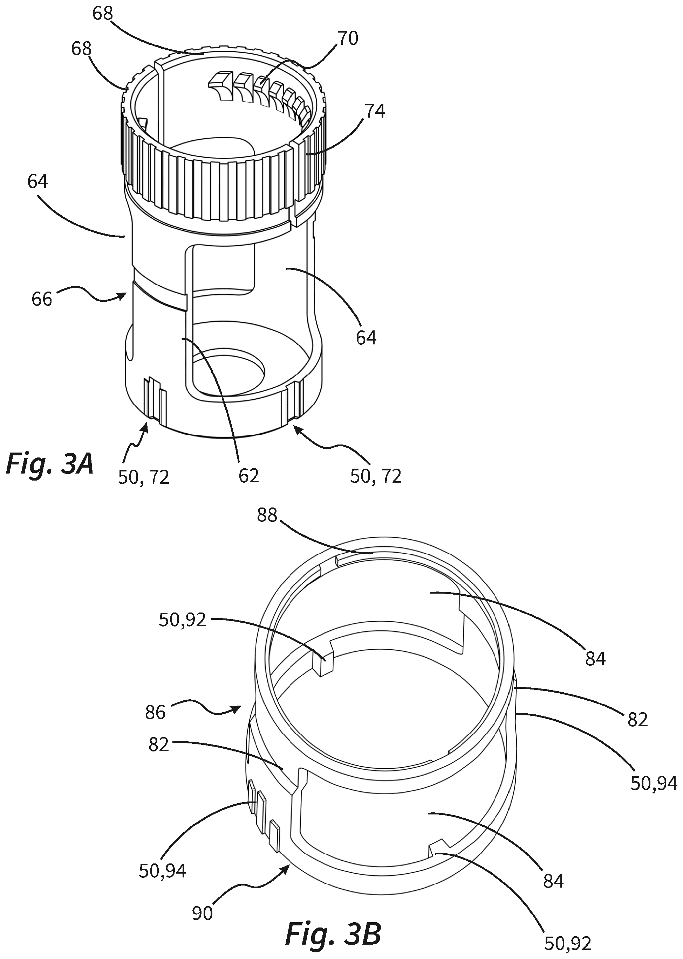

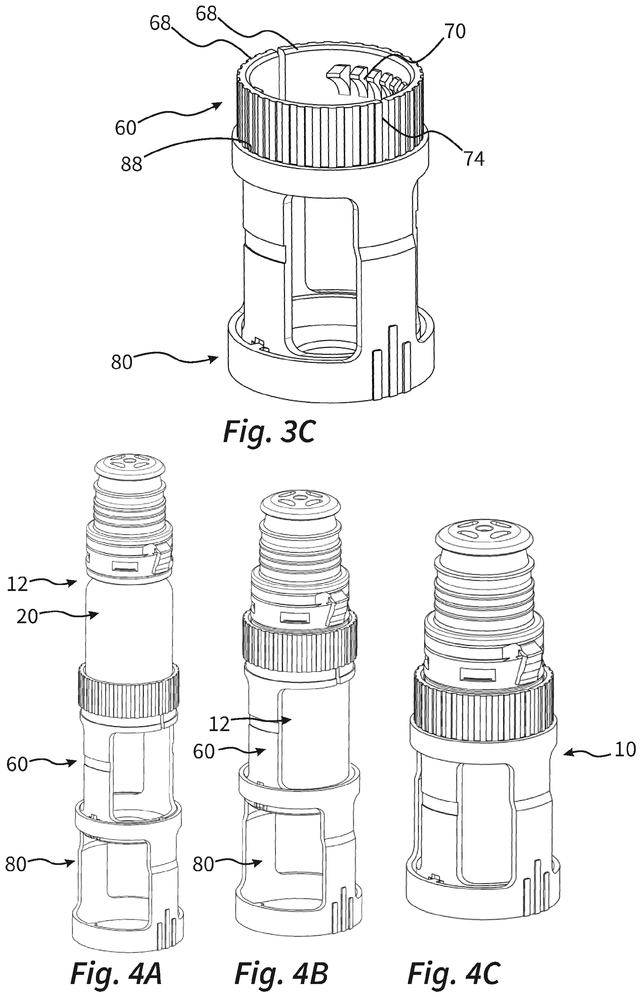

Die Fig. 3A bis 3C zeigen in jeweils separater sowie in montierter Darstellung die Blendenkörper 60, 80.

Fig. 3A zeigt den zweiten Blendenkörper 60, der beim vorliegenden Ausführungsbeispiel den inneren Blendenkörper bildet. Es ist zu erkennen, dass dessen Körper im Bereich des oberen Endes zweigeteilt ist. Zwei in etwa halbkreisförmige Sicherungsabschnitte 68 sind vorgesehen, deren Außenseite eine weitgehend umlaufende Grifffläche 74 bilden und die an der Innenseite eine Haltekontur 70 aufweisen. Durch die beschriebene Zweiteilung kann der zweite Blendenkörper 60 aufgeweitet werden, wenn er von unten auf den Quetschflaschenkörper 20 aufgeschoben wird. Weiterhin ist in Fig. 3A zu erkennen, dass insgesamt vier jeweils um 90° versetzte Vertiefungen vorgesehen sind, deren Flanken jeweils Verriegelungselemente 72 zum Zusammenwirken mit den Verriegelungselementen 92 des ersten Blendenkörpers bilden. Hierdurch ist eine Drehfixierung der Blendenkörper 60, 80 sowohl im gesicherten Zustand als auch im Nutzzustand möglich.

Bei dem in Fig. 3B dargestellten ersten Blendenkörper 80 ist in der Figur zu erkennen, dass ein oberer Ringabschnitt mit seiner Innenfläche eine Stützfläche bildet. Deren Innendurchmesser ist an den Außendurchmesser des zweiten Blendenkörpers unterhalb der Grifffläche 74 angepasst.

Dies führt dazu, dass im montierten Zustand der Blendenkörper 60, 80 die Stützfläche verhindert, dass die beiden halbkreisförmigen Sicherungsabschnitte 68 nach außen ausgelenkt werden. Hierdurch wird wiederum verhindert, dass sich der zweite Blendenkörper 60 nach Anbringung am Quetschflaschenkörper 20 von diesem lösen kann.

Fig. 4A bis 4C verdeutlichen die Montage. Wie Fig. 4A und 4B zeigen, wird zunächst die Austragvorrichtung 12, umfassend den Quetschflaschenkörper 20, in den zweiten Blendenkörper 60 eingeschoben. Hierbei werden die genannten Sicherungsabschnitte 68 ausgelenkt. Sobald der Quetschflaschenkörper 20 in der in Fig. 4B dargestellten Art vollständig im zweiten Blendenkörper aufgenommen ist, sind die Sicherungsabschnitte 68 wieder in ihre Ausgangslage zurückgekehrt und halten den Quetschflaschenkörper mittels der Haltekontur 70.

Im nächsten Schritt wird nun in der aus Fig. 4B und 4C ersichtlichen Weise der erste Blendenkörper 80 aufgeschoben. Seine Stützfläche 88 verrastet unterhalb der Grifffläche 74 des zweiten Blendenkörpers 60, so dass eine Aufweitung des zweiten Blendenkörpers 60 nicht mehr möglich ist. Gleichzeitig ist der erste Blendenkörper 80 hierdurch auch gegen axiales Abziehen gesichert.

Die Fig. 4A bis 4C zeigen eine Gestaltung, bei der die beiden Blendenkörper 60, 80 bereits beim Ausgangszustand der Montage in Fig. 4A miteinander gekoppelt sich und eine Vormontageeinheit bilden. Wenngleich dies bevorzugt wird, ist es auch möglich, die Blendenkörper 60, 80 erst während der Montage miteinander zu verbinden.

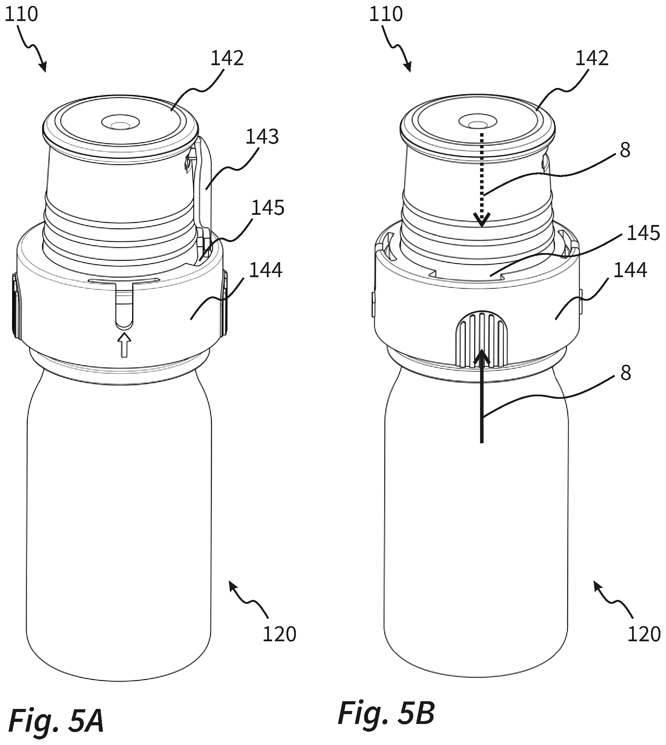

Fig. 5A bis Fig. 8. verdeutlichen den zweiten Erfindungsaspekt anhand eines zweiten Ausführungsbeispiels. Auch hier findet eine Austragvorrichtung 112 mit einem Austragkopf 130 und einem Quetschflaschenkörper 120 Verwendung. Die Austragvorrichtung 112 ist bis auf die Blendenkörper 60, 80 weitgehend identisch zu jener der Fig. 1 bis 4 und kann ebenfalls mit den beschriebenen Blendenkörpern 60, 80 ausgestattet sein. Übereinstimmend mit dem Ausführungsbeispiel der Fig. 1 bis 4 weist der Austragkopf eine Austragöffnung 132 auf, die von Tropfenbildungsmitteln 134 umgeben ist. Die Besonderheit der Gestaltung der Fig. 5 bis 8 liegt insbesondere in der Kappe 140. Diese verfügt über einen einstückigen Kappenkörper 142. An einem unteren Rand dieses Kappenkörpers 142 ist ein Verriegelungsring 144 aufgeschoben. Mittels eines umlaufenden Kragens 142A am Kappenkörper 142 und Rasten 144A am Verriegelungsring 144 sind der Kappenkörper 142 und der Verriegelungsring 144 dauerhaft miteinander verbunden. Die Verbindung ist jedoch ausreichend leichtgängig, dass der Verriegelungsring 144 im Nutzzustand frei gegenüber dem Kappenkörper 142 drehbar ist, insbesondere wenn der Kappenkörper 142 auf die Austragvorrichtung 112 klemmend aufgeschoben ist und daher selbst gegenüber der Austragvorrichtung 112 nicht oder kaum drehbar ist.

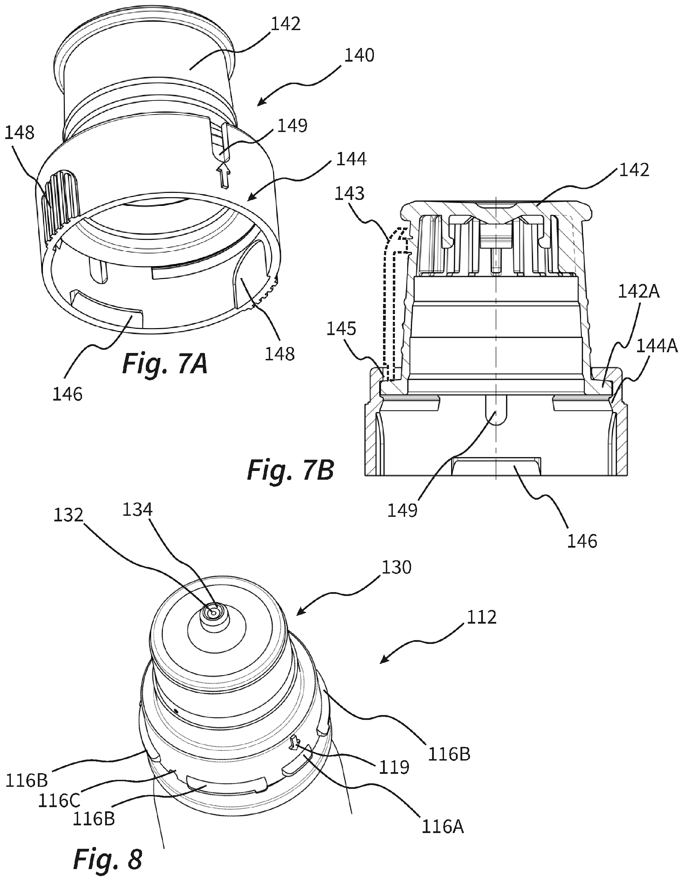

Im Lieferzustand der Fig. 5A ist die genannte Drehbarkeit noch nicht gegeben, da eine damit auch als Originalitätsabschnitt dienende Kunststoffzunge 143 in eine Aussparung 145 des Verriegelungsrings 144 ragt und dadurch eine Drehsicherung bildet. Zur Verdeutlichung ist Kunststoffzunge 143 gestrichelt auch in Fig. 7B dargestellt.

Erst wenn diese Kunststoffzunge 143 vom Kappenkörper 142 entfernt wurde, ist die Drehbewegung möglich. Dieser Nutzzustand bei bereits verdrehtem Verriegelungsring 144 ist in Fig. 5B gezeigt.

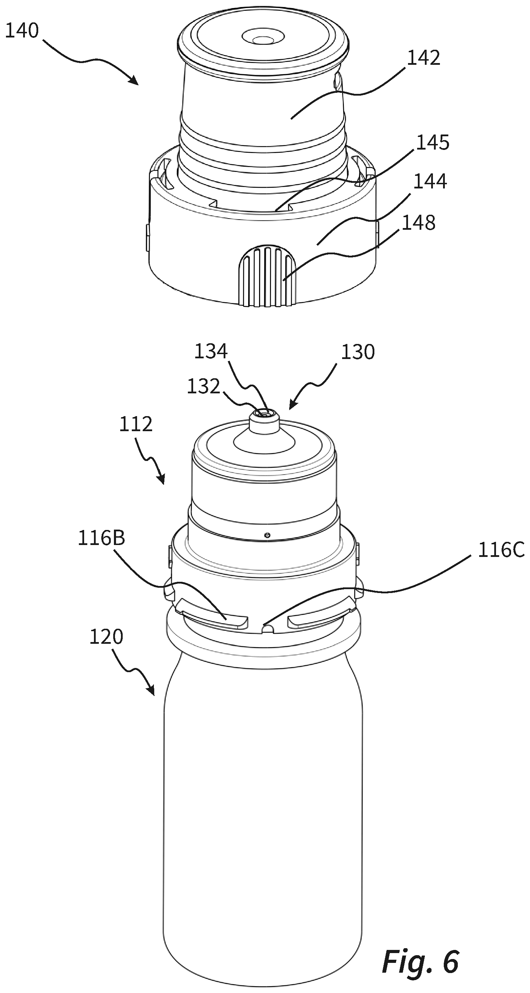

Fig. 6 zeigt den verwendungsbereiten Spender bei abgenommener Kappe 140.

Mittels des Verriegelungsrings 144 kann die Kappe 140 gesichert an der Austragvorrichtung 112 befestigt werden. Wie anhand der Fig. 7A und 7B ersichtlich ist, sind an der Innenseite des Verriegelungsrings 144 Verriegelungsprofile 146 in Form von nach innen weisenden Stegen mit Einführschrägen an der Unterseite vorgesehen. Korrespondierend hierzu sind auch am Austragkopf 130 Verriegelungsprofile 116A, 116B vorgesehen. Dies ist in Fig. 8 zu erkennen.

Ist die Kappe 140 auf die Austragvorrichtung 112 aufgesetzt, so untergreifen die Verriegelungsprofile 146 die Verriegelungsprofile 116A, 116B und verhindern ein einfaches Abziehen der Kappe 140.

Um die Kappe abzuziehen, müssen die Verriegelungsprofile 144 radial nach außen verlagert werden. Dies ist möglich, indem um 90° zu den Verriegelungsprofilen 146 versetzte Freigabe-Betätigungsflächen 148 in Richtung der Pfeile 8 aufeinander zu gedrückt werden, wie in Fig. 5B verdeutlicht ist.

Allerdings ist eine zum Lösen der Kopplung der Verriegelungsprofile 116A, 116B, 146 erforderliche Auslenkung der Verriegelungsprofile 146 nicht in jeder Drehstellung des Verriegelungsrings 144 möglich. Nur in der Drehstellung der Fig. 5B, in der die Freigabe-Betätigungsflächen 148 im Bereich einer Aussparung 116C zwischen den Verriegelungsprofilen 116B angeordnet sind und die Verriegelungsprofile 146 im Bereich der Verriegelungsprofile 116A angeordnet sind, ist eine ausreichende Verformung möglich.

Um die passende Ausrichtung erkennen zu können, sind Ausrichtungsmarkierungen 119, 149 vorgesehen. Die Ausrichtungsmarkierung 119 an der Austragvorrichtung 112 besteht in einem Symbol, vorliegend einem Pfeil-Symbol, auf einer Mantelfläche des Austragkopfes 130. Die Ausrichtungsmarkierung 149 der Kappe umfasst eine Durchbrechung, durch die bei passender Ausrichtung die Ausrichtungsmarkierung 119 am Austragkopfes 130 von außen erkennbar ist.

Während beim Abnehmen der Kappe 140 somit zunächst der Verriegelungsring 144 in die passende Ausrichtung zu bringen ist, kann je nach Gestaltung der Kappe beim Aufsetzen hierauf verzichtet werden. Dies wird durch passende Einführschrägen an mindestens einem derVerriegelungsprofilen 116A, 116B, 146 erreicht.

Nachfolgend sind noch weitere Ausführungsbeispiele dargestellt:
Beispiel 1. Flüssigkeitsspender zur Abgabe pharmazeutischer Flüssigkeiten mit den folgenden Merkmalen:
   a. der Flüssigkeitsspender weist einen Quetschflaschenkörper mit einem Behälterhals auf, und
   b. der Flüssigkeitsspender weist einen am Quetschflaschenkörper befestigten Austragkopf auf, der über eine Austragöffnung verfügt, durch die hindurch Flüssigkeit aus dem Quetschflaschenkörper austragbar ist, und
   c. der Flüssigkeitsspender weist einen ersten Blendenkörper auf, der mindestens eine Sperrwandung aufweist und mindestens eine Betätigungsaussparung definiert, und
   d. der erste Blendenkörper ist unverlierbar und beweglich am Flüssigkeitsspender vorgesehen, und
   e. der erste Blendenkörper überdeckt in einer Sperrstellung mittels der Sperrwandung einen Teilbereich des Quetschflaschenkörpers , so dass ein Zusammendrücken des Quetschflaschenkörpers verhindert oder zumindest erschwert ist, und gibt in einer Freigabestellung mittels der Betätigungsaussparung den Teilbereich des Quetschflaschenkörpers frei, so dass ein Zusammendrücken des Quetschflaschenkörpers ermöglicht ist.
Beispiel 2. Flüssigkeitsspender nach Beispiel 1 mit den folgenden weiteren Merkmalen:
   a. es ist ein zweiter Blendenkörper vorgesehen,
   b. der zweite Blendenkörper weist mindestens eine Sperrwandung auf und definiert mindestens eine Betätigungsaussparung, und
   c. der erste Blendenkörper ist gegenüber dem zweiten Blendenkörper verlagerbar, so dass in der Freigabestellung die Betätigungsaussparungen derart überlappen, dass durch beide Betätigungsaussparungen hindurch eine Kraftbeaufschlagung des Quetschflaschenkörpers ermöglicht ist, und so dass in der Sperrstellung die Betätigungsaussparungen derart gegeneinander versetzt sind, dass eine Betätigung des Quetschflaschenkörpers verhindert oder zumindest erschwert ist,
   vorzugsweise mit dem folgenden zusätzlichen Merkmal:
   d. der zweite Blendenkörper weist eine zumindest annähernd umlaufende Grifffläche auf, die vorzugsweise mit einer Strukturierung, insbesondere durch ein regelmäßiges Muster von Erhöhungen oder Vertiefungen gebildet ist.
Beispiel 3. Flüssigkeitsspender nach einem der vorstehenden Beispiele mit dem folgenden weiteren Merkmal:
   a. der erste Blendenkörper ist drehbar gegenüber dem Quetschflaschenkörper und/oder gegenüber dem zweiten Blendenkörper , wobei die Drehachse vorzugsweise mit einer Mittelachse (2) des Quetschflaschenkörpers übereinstimmt,
   vorzugsweise mit mindestens einem der folgenden zusätzlichen Merkmale:
   b. der zweite Blendenkörper ist gegenüber dem Quetschflaschenkörper drehfest, insbesondere durch eine Klemmverbindung zwischen dem zweiten Blendenkörper und dem Quetschflaschenkörper, oder
   a. der zweiten Blendenkörper ist drehbar gegenüber dem Quetschflaschenkörper und gegenüber dem ersten Blendenkörper.
Beispiel 4. Flüssigkeitsspender nach einem der vorstehenden Beispiele mit dem folgenden weiteren Merkmal:
   a. der erste und/oder der zweite Blendenkörper weist einen im wesentlichen rotationssymmetrischen Hülsenabschnitt auf, in dem die mindestens eine Betätigungsaussparung in Form einer Durchbrechung vorgesehen ist,
   vorzugsweise mit mindestens einem der folgenden zusätzlichen Merkmale:
   b. es ist genau eine Durchbrechung am ersten bzw. am zweiten Blendenkörper vorgesehen, wobei vorzugsweise diese Durchbrechung in Umfangsrichtung einen Winkelbereich zwischen 120° bis 240° überspannt, insbesondere vorzugsweise einen Winkelbereich zwischen 150° und 210°, oder
   c. es sind zwei Durchbrechungen am ersten bzw. am zweiten Blendenkörper vorgesehen, die jeweils einen Winkelbereich zwischen 60° und 170° überspannen, vorzugsweise jeweils einen Winkelbereich zwischen 90° und 160°.
Beispiel 5. Flüssigkeitsspender nach einem der Beispiele 2 bis 4 mit dem folgenden weiteren Merkmal:
   a. die beiden Blendenkörper sind derart relativ zueinander angeordnet, dass die Sperrwandung des zweiten Blendenkörpers im gesperrten Zustand zwischen dem Quetschflaschenkörper und der Sperrwandung des ersten Blendenkörpers angeordnet ist.
Beispiel 6. Flüssigkeitsspender nach einem der Beispiele 2 bis 5 mit den folgenden weiteren Merkmalen:
   a. der zweite Blendenkörper weist mindestens einen elastisch auslenkbaren Sicherungsabschnitt auf, der ein Herausziehen des Quetschflaschenkörpers aus dem zweiten Blendenkörper verhindert, und
   b. der erste Blendenkörper weist eine Stützfläche auf, durch die die elastische Auslenkbarkeit des Sicherungsabschnitts des zweiten Blendenkörpers beschränkt ist,
   insbesondere vorzugsweise mit mindestens einem der folgenden zusätzlichen Merkmale:
   c. die Stützfläche liegt außenseitig am zweiten Blendenkörper im Bereich des elastisch auslenkbaren Sicherungsabschnitts am zweiten Blendenkörper an, und/oder
   d. es sind eine Mehrzahl von radial auslenkbaren Sicherungsabschnitten vorgesehen, wobei eine gemeinsame und vorzugsweise umlaufende Stützfläche die radiale Auslenkbarkeit beschränkt.
Beispiel 7. Flüssigkeitsspender nach einem der vorstehenden Beispiele mit dem folgenden weiteren Merkmal:
   a. es ist eine schaltbare Verriegelung vorgesehen, mittels derer der erste Blendenkörper in seiner Sperrstellung rotativ verriegelt werden kann,
   vorzugsweise mit mindestens einem der folgenden zusätzlichen Merkmale:
   b. der erste Blendenkörper ist in der Sperrstellung durch formschlüssig wirkende Verriegelungselemente rotativ verriegelbar, und/oder
   c. der erste Blendenkörper ist mittels der Verriegelung am zweiten Blendenkörper oder am Quetschflaschenkörper rotativ gesichert, und/oder
   d. mittels der schaltbaren Verriegelung kann der erste Blendenkörper auch in seiner Freigabestellung rotativ gesichert werden.
Beispiel 8. Flüssigkeitsspender nach einem der vorstehenden Beispiele mit dem folgenden weiteren Merkmal:
   a. der erste Blendenkörper weist ein elastisch verformbares umlaufendes Ringsegment auf, an welchem einander gegenüber außenliegende Entriegelungsflächen vorgesehen sind und an welchem innenliegend ein in Umfangsrichtung zwischen den Entriegelungsflächen mindestens ein Verriegelungselement vorgesehen ist.
Beispiel 9. Flüssigkeitsspender nach einem der vorstehenden Beispiele mit dem folgenden weiteren Merkmal:
   a. am ersten Blendenkörper oder am zweiten Blendenkörper ist ein elastisch auslenkbares Verriegelungselement vorgesehen, welches durch manuelle Kraftbeaufschlagung verlagerbar ist, insbesondere verschwenkbar, so dass es von einem korrespondierenden Verriegelungselement am anderen Blendenkörper oder am Quetschflaschenkörper getrennt wird.

## Patentansprüche

1. Flüssigkeitsspender (110) zur Abgabe pharmazeutischer Flüssigkeiten mit den folgenden Merkmalen:
a. der Flüssigkeitsspender (110) weist eine Austragvorrichtung (112) mit einen vorzugsweise durch einen Quetschflaschenkörper gebildeten Flüssigkeitsspeicher (120) und einem Austragkopf (130) mit Austragöffnung (132) auf, und
b. der Flüssigkeitsspender (110) weist eine wiederaufsetzbare Kappe (140) auf, die im aufgesetzten Zustand die Austragöffnung (132) überdeckt und im abgenommenen Zustand die Austragöffnung (132) zum Austrag von Flüssigkeit freigibt, und
c. die Kappe (140) weist einen Kappenkörper (142) und einen am Kappenkörper drehbar angebrachten Verriegelungsring (144) auf, und
d. an der Austragvorrichtung (112) und am Verriegelungsring (144) sind im aufgesetzten Zustand aufeinander zuweisende Verriegelungsprofile (116, 146), die in mindestens einer Sicherungs-Drehstellung des Verriegelungsrings (144) zur Austragvorrichtung (112) formschlüssig ein Abnehmen der Kappe (140) verhindern, und
e. die aufeinander zuweisende Verriegelungsprofile (116, 146) sind derart ausgebildet, dass ein Abnehmen der Kappe (140) nur möglich ist, wenn der Verriegelungsrings (144) zur Austragvorrichtung (112) in eine Freigabe-Drehstellung gedreht worden ist.

2. Flüssigkeitsspender (110) nach Anspruch 1 mit dem folgenden weiteren Merkmal:
die aufeinander zuweisenden Verriegelungsprofile (116, 146) sind derart ausgebildet, dass ein Abnehmen der Kappe (140) in der Freigabe-Drehstellung nur dann möglich ist, wenn zusätzlich der Verriegelungsring (144) oder ein Teilabschnitt der Austragvorrichtung (112) elastisch verformt wird.

3. Flüssigkeitsspender (110) nach einem der Ansprüche 1 oder 2 mit dem folgenden weiteren Merkmal:
der Verriegelungsrings (144) weist auf einander gegenüberliegenden Seiten Freigabe-Betätigungsflächen (148) auf, die in der Freigabe-Drehstellung bei gleichzeitiger Kraftbeaufschlagung radial aufeinander zu die das Verriegelungsprofil (146) derart radial nach außen verlagern, dass anschließend ein Abnehmen der Kappe (140) möglich ist.

4. Flüssigkeitsspender (110) nach einem der Ansprüche 1 bis 3 mit dem folgenden weiteren Merkmal:
der Kappenkörper (142) und der Verriegelungsring (144) verfügen über zusammenwirkende Rastmittel (142A, 144A), mittels derer der Kappenkörper (142) und der Verriegelungsring (144) unter Gewährleistung der Drehbeweglichkeit verrastbar sind, wenn der Kappenkörper von unten in den Verriegelungsring eingeschoben wird.

5. Flüssigkeitsspender (110) nach einem der Ansprüche 1 bis 4 mit dem folgenden weiteren Merkmal:
der Verriegelungsring (144) und der Kappenkörper (142) weisen eine Formgebung auf, durch die sie im Lieferzustand drehgesichert miteinander verbunden, wobei ein Teilabschnitt (143) des Verriegelungsrings (144) oder des Kappenkörpers (142) entfernbar ist, um die Drehbarkeit herzustellen.

6. Flüssigkeitsspender (110) nach einem der Ansprüche 1 bis 5 mit dem folgenden weiteren Merkmal:
die Austragvorrichtung (112) und der Verriegelungsring (144) weisen Ausrichtungsmarkierungen (119, 149) auf, die übereinstimmend ausgerichtet sind, wenn der Verriegelungsring (144) sich in seiner Freigabestellung befindet.

7. Flüssigkeitsspender (110) nach einem der Ansprüche 1 bis 6 mit dem folgenden weiteren Merkmal:
die Ausrichtungsmarkierung (119) an der Austragvorrichtung (112) ist derart angeordnet, dass sie vom Verriegelungsring (144) verdeckt wird, wenn sich dieser nicht in der Freigabestellung befindet, wobei vorzugsweise im Verriegelungsring (144) eine Aussparung (149) vorgesehen ist, durch die hindurch die Ausrichtungsmarkierung (119) der Austragvorrichtung (112) sichtbar ist, wenn sich der Verriegelungsring in der Freigabestellung befindet.

8. Flüssigkeitsspender (10) nach einem der vorstehenden Ansprüche mit dem folgenden weiteren Merkmal:
der Flüssigkeitsspender (10) ist als Tropfer zur Abgabe von Einzeltropfen ausgebildet, insbesondere als Tropfer zur ophthalmischen Anwendung,

9. Flüssigkeitsspender (110) nach einem der vorstehenden Ansprüche mit dem folgenden weiteren Merkmal:
der Flüssigkeitsspender (10) verfügt über Tropfenbildungsmittel (34), insbesondere in Form einer planen oder konischen Tropfenbildungsfläche.
